# FASCICULE DE BREVET EUROPEEN

(11) **EP 3 069 755 B1**
(45) Date de publication et mention de la délivrance du brevet: **25.10.2017**
(21) Numéro de dépôt: 16155619.6
(22) Date de dépôt: 15.02.2016
(51) Int. Cl.: A61N 1/375, A61B 5/00, A61B 5/042, A61N 1/05, A61N 1/362

(54) **DISPOSITIF MÉDICAL IMPLANTABLE ACTIF COMPRENANT UNE CAPSULE DÉPOURVUE DE CONNECTEUR, RELIÉE DE FAÇON PERMANENTE À UNE MICROSONDE**
AKTIVE IMPLANTIERBARE MEDIZINISCHE VORRICHTUNG, DIE EINE KAPSEL OHNE ANSCHLUSS UMFASST, DIE PERMANENT MIT EINER MIKROSONDE VERBUNDEN IST
ACTIVE IMPLANTABLE MEDICAL DEVICE COMPRISING A CONNECTOR-FREE CAPSULE, PERMANENTLY CONNECTED TO A MICROPROBE

(30) Priorité: 18.03.2015 FR 1552211
(43) Date de publication de la demande: 21.09.2016
(73) Titulaire: Sorin CRM SAS, 92140 Clamart (FR)
(72) Inventeur: REGNIER, Willy, 91160 LONGJUMEAU (FR)
(74) Mandataire: Grünecker Patent- und Rechtsanwälte PartG mbB

(56) Documents cités:
- WO-A2-2013/080038
- US-A1- 2010 137 960
- US-A1- 2013 110 127
- US-A1- 2013 123 875

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Toutefois, bien que dans la suite on décrira principalement un ensemble de détection/stimulation cardiaque, cette application n'est pas limitative de l'invention qui, comme on le comprendra, peut être également appliquée, *mutatis mutandis,* à la détection/stimulation d'autres organes tels que système nerveux (notamment pour la stimulation du cerveau ou des nerfs), réseau artériel ou lymphatique, système digestif (estomac, intestin) ou encore le système respiratoire.

Dans le cas du coeur, l'invention vise plus particulièrement la situation des patients en insuffisance cardiaque (HF, *Heart Failure*), auxquels est proposée l'implantation d'un dispositif de resynchronisation cardiaque de type dit CRT-P (stimulateur cardiaque) ou CRT-D (stimulateur avec en outre une fonction de défibrillateur).

La thérapie vise à resynchroniser la contraction des deux ventricules entre eux, et si nécessaire celle des ventricules par rapport à l'oreillette, de manière à améliorer l'état du patient par optimisation des différentes phases du cycle hémodynamique. Pour cela, les dispositifs mettent en oeuvre une technique dite "CRT" (*Cardiac Resynchronisation Therapy*) ou "BVP" (*Bi-Ventricular Pacing*) consistant à délivrer en tant que de besoin des impulsions électriques assurant une stimulation conjointe et permanente des deux ventricules, gauche et droit, afin de resynchroniser les contractions de ces derniers.

En ce qui concerne le dispositif implanté, celui-ci nécessite l'implantation dans le ventricule droit d'une sonde endocavitaire conventionnelle de stimulation et, pour la stimulation du ventricule gauche, d'une sonde introduite dans le réseau veineux coronaire via le sinus coronaire, de manière à placer l'électrode de stimulation de cette sonde contre la paroi du ventricule gauche. Une alternative consiste à utiliser comme sonde ventriculaire gauche une sonde épicardique, introduite dans le sac péricardique et fixée sur la paroi externe du muscle cardiaque. Le dispositif met également souvent en oeuvre une troisième sonde, placée dans la cavité auriculaire droite, permettant de détecter la contraction de l'oreillette afin de synchroniser sur celle-ci la stimulation des ventricules, en respectant la chronologie du délai atrioventriculaire.

Ces sondes, qu'elles soient endocavitaires ou coronaires, sont introduites via le réseau veineux du patient, ce qui peut créer des complications telles que déplacement, rupture d'isolant ou de conducteur, développement de fibroses, etc.

Pour réduite ces risques, une nouvelle génération de dispositifs a été développée, qui se présentent sous forme de capsules autonomes implantables dans une cavité cardiaque (ventricule, oreillette ou même cavité cardiaque gauche artérielle), et sont généralement désignées "capsules *leadless*". Ces capsules sont dépourvues de toute liaison physique à un dispositif principal implanté (tel qu'un boitier de générateur d'impulsions de stimulation) ou non implanté (périphérique externe tel que programmateur ou dispositif de *monitoring* pour le suivi à distance du patient). Elles sont qualifiées pour cette raison de *leadless,* pour les distinguer des électrodes disposées à l'extrémité distale d'une sonde (*lead*) conventionnelle, parcourue sur toute sa longueur par un ou plusieurs conducteurs reliant par voie galvanique l'électrode distale à un connecteur situé à l'extrémité opposée, proximale, de la sonde, ce connecteur étant destiné à être branché sur le boitier du générateur d'impulsions.

Le WO 2013/080038 A2 décrit un exemple d'une telle capsule *leadless* endocavitaire ou épicardique, et ses moyens de communication avec l'environnement extérieur. Le dispositif comprend également un générateur d'impulsions implanté en sous-cutané, avec une liaison filaire à une électrode endocavitaire ou épicardique placée en un site de détection/stimulation. La synchronisation de l'ensemble est assurée par une liaison sans fil entre les capsules *leadless* et le générateur maitre distant.

Ces capsules *leadless* peuvent avantageusement remplacer les sondes endocavitaires conventionnelles telles que les sondes ventriculaires droites et auriculaires droites, ou encore les sondes épicardiques, mais compte tenu de leur taille elles ne peuvent pas se substituer aux sondes de stimulation du ventricule gauche introduites dans le réseau coronaire veineux, sondes qui sont nécessaires pour la détection/stimulation du ventricule gauche, donc pour l'application d'une thérapie CRT. De plus, le réseau artériel endocavitaire (donnant donc accès aux cavités gauches) demeure toujours extrêmement risqué, même avec une capsule *leadless,* du fait des risques graves d'hémorragie ou de formation de caillots, susceptibles de former des emboles artériels.

D'autre part, en ce qui concerne les sondes coronaires gauches, la nécessité d'utiliser pour leur implantation un fil-guide de pose, la norme standard des connecteurs de sonde gauche multipolaires (normes IS-4 ou DF-4), ainsi que la nécessité d'une lumière centrale ménagée dans le corps de sonde pour l'introduction du fil-guide, sont des contraintes qui limitent la possibilité de réduire le diamètre de ces sondes coronaires et donc d'atteindre de nouvelles zones cibles de stimulation du ventricule gauche qui restent difficilement atteignables aujourd'hui.

On entend ici par "capsule hybride" un dispositif notamment pour la détection/stimulation du ventricule gauche, constitué :
- d'un corps pouvant être de même forme et de même configuration qu'une capsule *leadless*, avec une architecture électronique basse consommation, une source d'énergie miniaturisée et des moyens de communication sans fil avec d'autres capsules,
- où ce corps est pourvu d'une sonde prolongeant directement le corps de la capsule sans solution de continuité (c'est-à-dire sans connecteur intermédiaire), de manière à former un dispositif monobloc et entièrement autonome,
- cette sonde étant de surcroit de type "microsonde", c'est-à-dire une sonde miniaturisée de très faible diamètre (typiquement au plus 1 French, soit 0,33 mm) et dépourvue de lumière interne, formée d'un câble de coeur revêtu d'une couche d'isolement avec, en région distale, une ou plusieurs zones sélectivement dénudées formant électrodes de détection/stimulation.

Un but de l'invention est d'assurer, entre la sonde et le corps de capsule dans un tel dispositif, une connexion mécanique et électrique qui soit robuste, compacte et fiable.

L'invention propose à cet effet un dispositif médical implantable actif du type général divulgué par le WO 2013/080038 A2 précité, c'est-à-dire formé d'une capsule hybride autonome comprenant un corps étanche logeant des circuits électroniques et des moyens d'alimentation électrique de ces circuits électroniques et, prolongeant ce corps, au moins une sonde de détection et/ou de délivrance de thérapie, cette sonde comprenant un ensemble de câbles formés chacun d'un câble de coeur électriquement conducteur relié à un pôle respectif desdits circuits électroniques et au moins une électrode de détection et/ou de délivrance de thérapie. De façon caractéristique, la sonde est une microsonde comprenant un ensemble de microcâbles, le dispositif est dépourvu de connecteur électrique entre la microsonde et les circuits électroniques, chaque câble de coeur étant relié à son pôle respectif par des moyens de liaison permanente, et les moyens de liaison permanente comprennent :
- une embase isolante traversée de façon hermétique par un ensemble de broches correspondant aux pôles respectifs des circuits électroniques ;
- un support recevant l'embase de façon hermétique ;
- un ensemble de pièces conductrices de jonction, chaque pièce de jonction comprenant un premier passage pour un câble de coeur respectif et un second passage pour une broche respective et étant soudée sur ces derniers ;
- une pièce isolante de guidage et de retenue montée sur le support et verrouillée mécaniquement sur celui-ci, ladite pièce de guidage et de retenue comportant un ensemble de cavités pour les pièces de jonction respectives, chaque cavité s'ouvrant en son fond sur un passage dans lequel fait saillie une broche respective ; et
- au moins un élément de transition et de protection encapsulant la pièce de guidage et de retenue, les pièces de jonction et des régions des câbles de coeur qui leur sont attenantes.

Selon diverses caractéristiques subsidiaires avantageuses :
- le support fait partie du corps de la capsule ;
- le support est constitué d'une pièce formant face avant par laquelle les moyens de liaison sont fixés sur le corps de la capsule ;
- la pièce de guidage et de retenue est montée sur le support par un mouvement de translation suivi d'un mouvement de rotation assurant la mise en correspondance de languettes de verrouillage prévues respectivement sur la pièce de guidage et de retenue et sur le support ;
- dans ce dernier cas, au moins certaines languettes peuvent former une rampe assurant une mise en pression des languettes entre elles lors de la rotation, et/ou la pièce de guidage et de retenue peut comprendre un corps principal portant des languettes de verrouillage et guidant ladite pièce dans le support, et un corps secondaire plus étroit à la périphérie duquel sont formées les cavités ;
- les pièces de jonction comprennent chacune un corps principal dans lequel est formé le passage pour la broche respective, et un appendice s'étendant latéralement par rapport audit passage pour la broche et dans lequel est formé le passage pour le câble de coeur ;
- l'élément de transition et de protection comprend une région de matériau souple assurant l'encapsulation et dont la section transversale diminue progressivement du corps de la capsule vers la microsonde ;
- l'élément de transition comprend, autour de la région proximale de la microsonde attenante au corps de la capsule, une région de transition à gradient de raideur variable, décroissant dans le sens distal ;
- dans ce dernier cas, la région de transition peut notamment être formée d'au moins une pièce distincte de la région d'encapsulation et comprend à son extrémité proximale un aménagement de verrouillage dans la région d'encapsulation ;
- le corps de la capsule présente une forme généralement allongée de section transversale essentiellement constante, et dans lequel les moyens de liaison sont prévus à une extrémité du corps et occupent une section transversale au plus égale à celle du corps.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 montre, en situation d'implantation, les différents éléments constituant un ensemble CRT selon l'état de la technique.
La Figure 2 montre, en situation d'implantation, les différents éléments constituant un ensemble CRT selon l'invention.
La Figure 3 est une vue agrandie de la capsule hybride de l'ensemble de

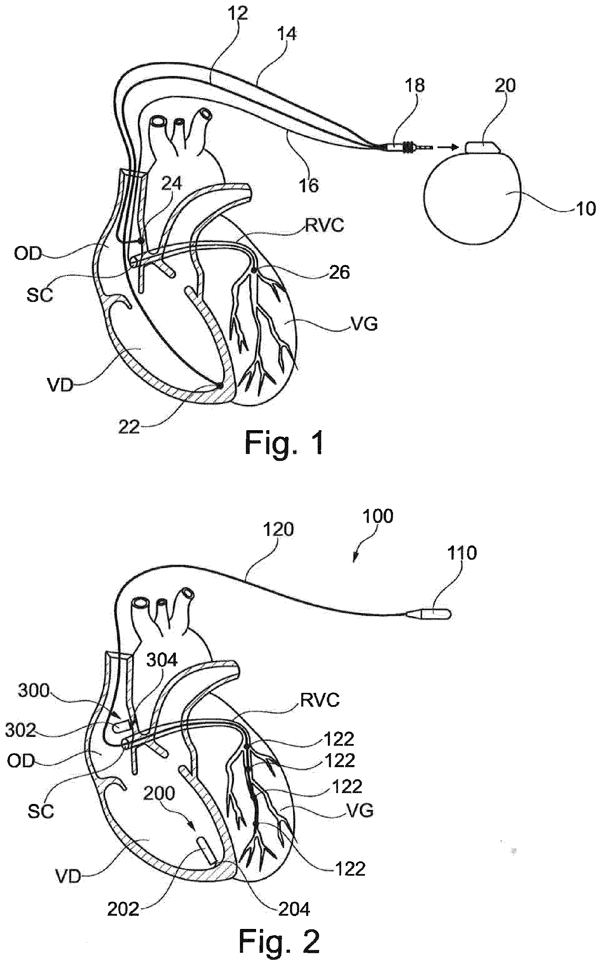

la Figure 2.

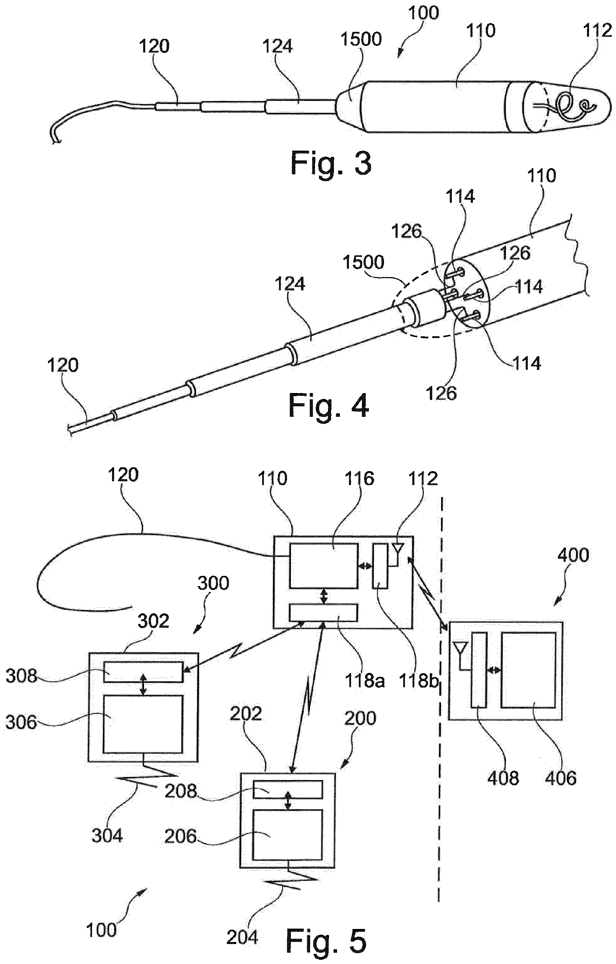

La Figure 4 est un détail montrant schématiquement la manière dont est réalisée la liaison électrique des conducteurs de la microsonde aux circuits internes de la capsule hybride.
La Figure 5 est un schéma fonctionnel décrivant les interactions entre les différents éléments de l'ensemble de l'invention.

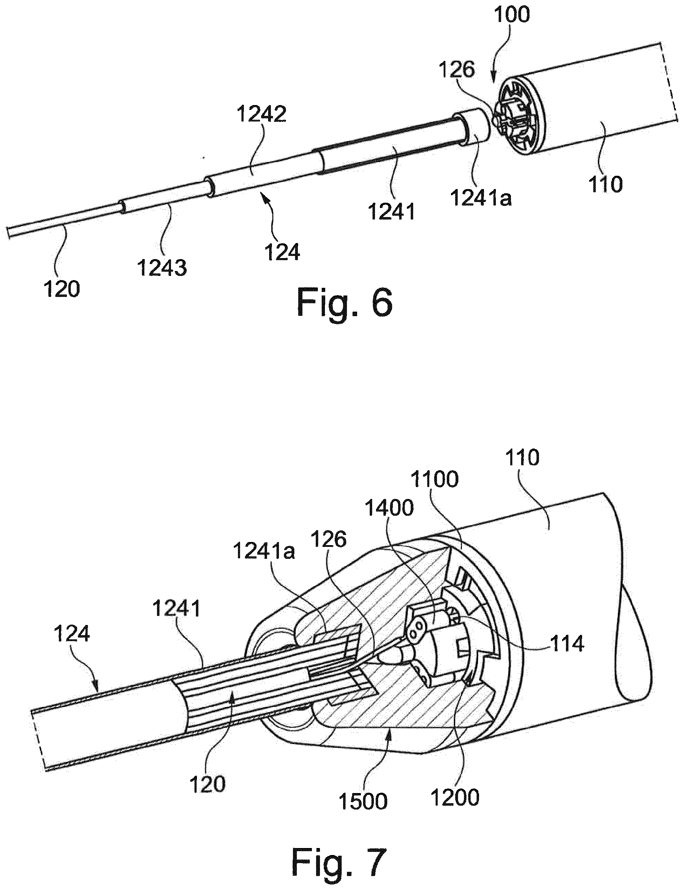

La Figure 6 est une vue en perspective éclatée d'une partie des composants de la liaison entre la capsule hybride et la microsonde.
La Figure 7 est une vue en perspective partielle de ladite liaison.

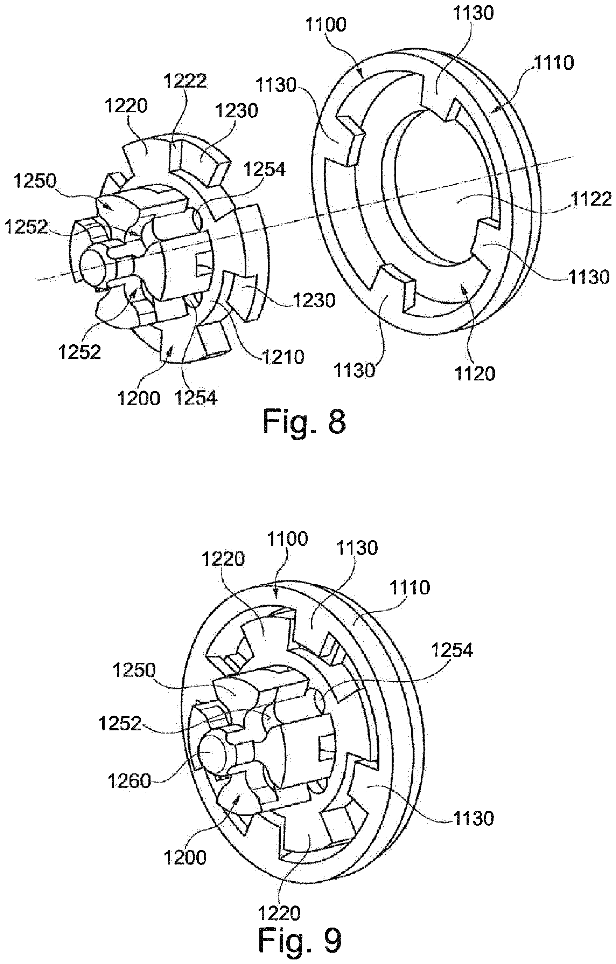

La Figure 8 est une vue en perspective éclatée de deux composants de ladite liaison.
La Figure 9 est une vue en perspective des deux composants dans un état assemblé.

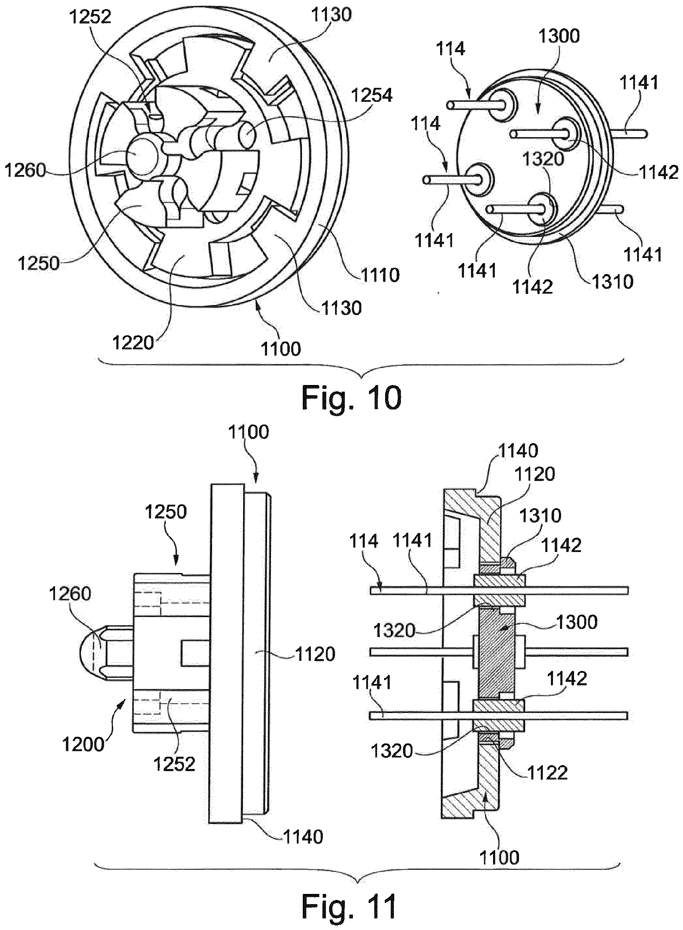

La Figure 10 est une vue en perspective éclatée de l'assemblage de la Figure 9 et d'un troisième composant de la liaison.
La Figure 11 est une vue en coupe axiale du troisième composant de la liaison et représente également l'assemblage de la Figure 9 en élévation de côté.

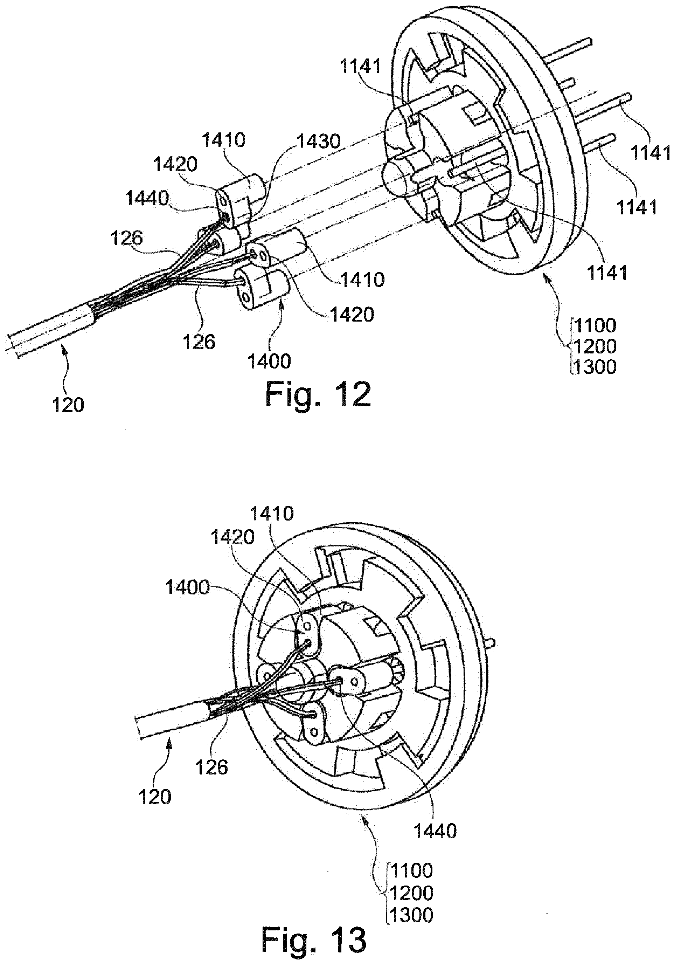

La Figure 12 est une vue en perspective éclatée de l'assemblage des trois composants des Figures 10 et 11 avec la microsonde et avec des pièces de jonction associées.
La Figure 13 est une vue en perspective des éléments de la Figure 12, assemblés.

On va maintenant décrire un exemple de réalisation de l'invention, appliquée à un ensemble de type resynchroniseur cardiaque (CRT).

Comme cela a été expliqué en introduction, cet exemple ne revêt qu'un caractère illustratif, l'invention pouvant être mise en oeuvre dans le cadre de configurations très variées de détection/stimulation, de surcroit dans un contexte qui n'est pas nécessairement lié à un diagnostic et/ou à une thérapie cardiaque.

La Figure 1 montre, en situation d'implantation, les différents éléments constitutifs d'un ensemble CRT selon l'état de la technique.

Cet ensemble comporte un générateur 10 de stimulateur CRT, par exemple de la famille *Paradym CRT* de Sorin CRM, Clamart, France. Ce générateur se présente sous forme d'un boitier de volume 30 cm³ environ auquel sont couplées trois sondes 12, 14 et 16 au moyen d'un connecteur 18 enfiché dans une tête de connecteur 20 du générateur 10, typiquement un connecteur de type normalisé IS-4. Le générateur 10 comprend une pile de longue durée pour l'alimentation des circuits internes de commande et de détection/stimulation, dont la puissance moyenne consommée est de l'ordre de 30 µW.

Les sondes de l'ensemble CRT comprennent une sonde ventriculaire droite 12 de type endocavitaire introduite dans le réseau veineux, comprenant un corps de sonde de diamètre typique 4 French (1,33 mm), terminé à son extrémité distale par une tête de sonde portant une électrode de détection/stimulation 22 ancrée au fond de la cavité du ventricule droit VD.

L'ensemble peut également (de manière optionnelle) comprendre une sonde auriculaire droite 14 de type endocavitaire, de structure comparable à celle de la sonde 12 avec un corps de sonde terminé à son extrémité distale par une tête de sonde implantée dans l'oreillette droite OD et pourvue d'une électrode de détection auriculaire 24.

Pour la détection/stimulation du ventricule gauche il n'est pas possible, ou tout au moins extrêmement risqué, d'utiliser une sonde endocavitaire, et pour cette raison on utilise généralement comme sonde ventriculaire gauche 16 une sonde introduite dans le réseau veineux coronaire SVC via le sinus coronaire SC débouchant dans le ventricule droit. Cette sonde coronaire 16 est pourvue à son extrémité distale d'une électrode 26 positionnée en appui contre la paroi du ventricule gauche VG de manière à pouvoir stimuler celui-ci dans la zone de cette électrode. En variante, la sonde ventriculaire gauche 16 peut être une sonde épicardique, introduite entre la paroi du myocarde et le sac épicardique entourant ce dernier, de manière à venir, de la même façon, en contact avec la paroi externe du muscle ventriculaire à stimuler.

La Figure 2 est homologue de la Figure 1, mais pour un ensemble CRT 100 selon l'invention.

Cet ensemble comporte, en lieu et place du générateur 10, un dispositif ci-après dénommé "capsule hybride" 100, associé à une unique sonde 120 couplée au corps 110 de la capsule hybride par un système de liaison simplifié, permanent, remplaçant un connecteur tel que le connecteur IS-4 18 de l'ensemble selon l'état de la technique illustré Figure 1. En d'autres termes, la sonde 120 prolonge le corps 110 de la capsule sans solution de continuité du fait de l'absence de connecteur.

La sonde 120 est une sonde de type "microsonde" telle que décrite notamment dans le EP 2 719 422 A1 (Sorin CRM). Il s'agit d'une sonde de très faible diamètre dans sa partie distale, typiquement un diamètre inférieur à 1,5 French (0,5 mm), de préférence d'au plus 1 French (0,33 mm). Cette sonde est réalisée à partir d'au moins un microcâble constitué lui-même d'un câble de coeur électriquement conducteur revêtu d'une couche d'isolement entourant le câble de coeur et présentant au moins une zone sélectivement dénudée ménagée dans la couche d'isolement pour former une électrode de détection/stimulation. Diverses structures de microcâbles sont notamment décrites dans le EP 2 581 107 A1 (Sorin CRM) auquel on pourra se référer pour de plus amples détails. Avantageusement, comme décrit dans le EP 2 719 422 A1 précité, une pluralité de ces microcâbles sont assemblés ensemble en un toron de microcâbles, chacun électriquement indépendant, de manière à obtenir une microsonde multipolaire dotée de plusieurs électrodes 122 sélectionnables distinctement. Une telle microsonde multipolaire permet notamment de mettre en oeuvre une fonction dite de "repositionnement électrique" consistant à sélectionner, parmi plusieurs points de stimulation correspondant à plusieurs électrodes respectivement reliées à l'un des microcâbles de la sonde, celui assurant la meilleure efficacité. Cette sélection peut être opérée aussi bien au moment de l'implantation de la sonde qu'ultérieurement, en effectuant à intervalles réguliers des tests pour vérifier que le site initialement choisi est toujours optimal, et éventuellement en sélectionner un autre dans le cas contraire.

La partie distale, active, de la microsonde 120 est implantée dans le réseau veineux coronaire RVC de manière que les électrodes 122 soient en contact avec différentes zones de la paroi du ventricule gauche VG. Les diverses électrodes 122 peuvent être constituées par une pluralité de régions dénudées d'une zone monopolaire (ces électrodes étant donc toutes actives et reliées électriquement en parallèle), ou bien par des électrodes différentes, sélectivement commutables, d'une microsonde multipolaire.

L'ensemble de l'invention comporte en outre une capsule *leadless* 200 implantée dans le ventricule droit VD. Cette capsule est de type *leadless,* c'est-à-dire qu'elle est dépourvue de toute liaison physique à un dispositif principal implanté (tel que le générateur 10 de la Figure 1) ou non implanté. Une telle capsule *leadless* comporte un corps 202 muni à l'une de ses extrémités d'un organe d'ancrage 204, généralement une vis hélicoïdale saillante prolongeant axialement le corps 202 de la capsule, et destiné à pénétrer dans le tissu cardiaque par vissage au site d'implantation, de la même manière que pour les sondes à vis conventionnelles.

Le EP 2 394 695 A1 (Sorin CRM) décrit un tel type de capsule *leadless* à vis, ainsi qu'un accessoire permettant son implantation au site choisi par accostage de la vis axiale, entrainement en rotation de la capsule pour fixer celle-ci de façon permanente à la paroi cardiaque où elle se trouvera maintenue par la vis axiale d'ancrage, puis retrait de l'accessoire, la capsule restant alors librement fixée à la paroi cardiaque.

Le corps 202 d'une telle capsule *leadless* se présente habituellement sous une forme générale cylindrique avec une longueur de 20 à 40 mm, un diamètre extérieur inférieur à 6 mm (2 French, dimension imposée par le calibre de la voie d'accès via le réseau veineux périphérique), et un volume d'environ 1 cm³.

La capsule *leadless* intègre une architecture électronique à basse consommation, typiquement 5 à 8 µW, ce qui permet de l'alimenter par un système de récupération d'énergie ou *harvester* (décrit par exemple dans les EP 2 638 930 A1 (Sorin CRM) ou EP 2 639 845 A1 (Sorin CRM)) en lieu et place d'une pile dont la durée de vie est par nature limitée.

Dans la configuration illustrée Figure 2, il est également prévu une seconde capsule *leadless* 300 d'un type comparable à la capsule 200, comprenant un corps 302 et des moyens 304 d'ancrage à la paroi cardiaque, cette paroi étant celle de l'oreillette droite OD de manière à pouvoir recueillir les signaux de dépolarisation auriculaires.

Les capsules 200 et 300 sont des capsules *leadless* conventionnelles, d'un type en elles-mêmes connu, et ne seront pas décrites plus en détail pour cette raison.

Les Figures 3 et 4 sont des vues agrandies montrant des détails de la capsule hybride 100 de l'ensemble selon l'invention.

La capsule hybride comprend un corps tubulaire métallique 110 (en titane ou alliage) étanche, atraumatique et biocompatible. Le diamètre externe de ce corps est d'au plus 6 mm (18 French), sa longueur est d'au plus 40 mm, et son volume est de l'ordre de 1 cm³. En d'autres termes, le corps de la capsule hybride 110 a sensiblement les mêmes dimensions qu'une capsule *leadless* conventionnelle telle que les capsules 200 et 300.

À l'une des extrémités, le corps 110 de la capsule hybride est pourvu d'une antenne 112 pour une communication sans fil, notamment pour lui permettre de communiquer avec un périphérique externe de type programmateur ou dispositif de télétransmission de données, notamment par télémétrie RF dans la bande MICS (*Medical Implant Communication Sys-tem*), MEDS, dans les bandes banalisées publiques ISM utilisées par les dispositifs médicaux, ou encore par communication selon les protocoles *Bluetooth.*

Pour la communication HBC entre capsules, il peut être prévu une électrode en forme d'anneau, isolée électriquement du corps 110 de la capsule et de l'antenne 112, destinée à assurer la transmission de données par contact avec les tissus ou le sang via des impulsions électriques dans le corps du patient.

À l'extrémité opposée, le corps 110 de la capsule hybride 100 est prolongé par la microsonde 120, avec une région intermédiaire 124 de transition procurant, sur une longueur de l'ordre de 30 mm, un gradient de raideur progressif entre l'extrémité rigide du corps 110 et la partie souple de la microsonde 120.

Comme illustré Figure 4, la liaison entre les microcâbles 126 de la microsonde 120 et les circuits électriques internes contenus dans le corps 110 de la capsule hybride est réalisée par des traversées 114 étanches, qui sont des traversées unipolaires avec une broche conductrice soudée sur une embase solidaire du corps 110 de la capsule hybride et reliées chacune à un microcâble 126 respectif de la microsonde 120.

La Figure 5 est un schéma fonctionnel illustrant les interactions entre les différents dispositifs de l'ensemble que l'on vient de présenter.

Chacune des capsules hybride 100 ou *leadless* 200 et 300 comporte des circuits électroniques de commande, respectivement 116, 206, 306, couplés à des moyens émetteurs/récepteurs de communication sans fil, respectivement 118a-118b, 208 et 308, pour permettre la communication mutuelle entre les différentes capsules 100, 200, 300 ainsi que pour la communication de la capsule hybride 110 avec un équipement distant 400. L'équipement distant 400 comprend de son côté des circuits 406 couplés à des moyens émetteurs/récepteurs 408. Cet équipement externe 400 peut être notamment le programmateur d'un praticien, la communication servant alors à interroger l'ensemble implanté, y lire des données stockées en mémoire, en modifier certains paramètres, etc., ou encore un dispositif de *home monitoring* pour le suivi à domicile de l'état du patient avec possibilité de transmission des informations à un site distant, hospitalier ou autre.

On va maintenant décrire en détail, en référence aux Figures 6 à 13, une solution préférée pour réaliser la liaison entre les microcâbles 126 de la microsonde 120 et les circuits électriques internes de la capsule hybride via les traversées 114.

Cette liaison fait appel à une première pièce 1100 formant face avant du corps 110 de la capsule hybride et à une pièce support de liaison 1200 qui supporte les fonctions de positionnement, maintien, blocage de l'ensemble des composants intervenant dans la liaison.

La pièce formant face avant 1100 comprend une partie de base 1120 en forme de disque plan pour son appui en direction axiale contre l'extrémité avant de la capsule. Cette partie de base est traversée par une ouverture centrale 1122 pour la fixation d'une embase 1300 illustrée sur les Figures 10 et 11 et que l'on décrira dans la suite. La partie de base 1120 est prolongée axialement par une collerette périphérique annulaire 1110 à partir de laquelle s'étendent vers l'intérieur un ensemble de languettes de verrouillage 1130 (ici au nombre de quatre) destinées à coopérer avec des organes de verrouillage homologues de la pièce de liaison.

Comme le montre la Figure 7, la pièce formant face avant 1100 présente des dimensions qui correspondent aux dimensions transversales de la capsule hybride de manière à ne pas augmenter l'encombrement de la capsule.

La pièce support de liaison 1200 comprend un corps principal 1210 de section transversale généralement circulaire à partir duquel s'étendent vers l'extérieur quatre secteurs 1220. À partir de chaque secteur s'étend, dans une direction circonférentielle et sur une partie seulement de son épaisseur, une languette de verrouillage 1230.

Comme cela est représenté en détail Figures 8 et 9, les languettes 1230 sont destinées à coopérer avec les languettes 1130 de la partie 1100 formant face avant selon un principe de montage "à baïonnette", la pièce 1200 étant engagée par translation axiale dans la cavité définie par la collerette 1110 de la pièce 1100 de telle sorte que les secteurs 1220 munis de leur languette respective 1230 passent entre deux languettes adjacentes 1130, puis tournée d'un angle déterminé autour de l'axe principal de la capsule, dans le sens horaire sur la Figure 8, de façon à ce que les languettes 1230 de la pièce 1200 viennent se verrouiller en arrière des languettes 1130 de la pièce 1100 en maintenant fermement les deux pièces solidaires l'une de l'autre.

La fermeté de ce verrouillage peut être obtenue en donnant aux languettes 1130 et/ou 1230, au niveau de leurs surfaces qui viennent en contact mutuel, une pente de quelques degrés, par exemple de 3°, un serrage progressif étant ainsi obtenu avec mise en pression progressive des languettes entre elles, jusqu'à la rotation maximum définie par la venue en butée de chaque languette 1130 contre l'épaulement 1222 existant entre la languette homologue 1230 et le secteur 1220 qui la porte.

La pièce support de liaison 1200 comporte par ailleurs une superstructure formée par un corps secondaire 1250 de contour généralement cylindrique, coaxial avec le corps principal 1210 mais de diamètre plus petit. Ce corps secondaire abrite quatre cavités généralement cylindriques 1252 réparties à sa périphérie et dont les formes sont généralement complémentaires de celles de quatre pièces de jonction respectives 1400, associées aux microcâbles 126, telles qu'on les décrira plus loin. Ces cavités 1252 se prolongent dans des ouvertures traversantes 1254 traversant le corps principal 1210 vers l'arrière (vers la droite sur la Figure 8 par exemple).

La pièce 1200 se termine, du côté du corps secondaire 1250, par une proéminence axiale 1260 permettant d'assurer un préguidage des pièces de jonction 1400.

La pièce 1100 est réalisée en matériau électriquement isolant et biocompatible, par exemple en polyétheréthercétone (PEEK) ou en un élastomère thermoplastique de polyuréthane de type *Tecothane* ou *Pellethane* (marques déposées).

La pièce 1200 est réalisée par exemple en alliage de titane, de manière à pouvoir être soudée par laser sur le corps 110 de la capsule, également de préférence en titane en ce cas.

On réalise grâce à la configuration décrite ci-dessus un assemblage extrêmement compact entre les pièces 1100 et 1200, à la fois en termes de diamètre (égal à celui de la capsule et typiquement de 5 à 7mm) et de longueur axiale (typiquement de 3 à 4 mm).

Comme le montrent bien les Figures 10 et 11, les traversées étanches 114 assurant la liaison électrique entre les microcâbles 126 de la microsonde 120 sont ici quatre traversées unipolaires qui sont montées dans l'embase 1300, constituée par une pièce en forme générale de disque qui vient se loger par l'arrière dans l'ouverture 1122 de la pièce formant face avant. Un épaulement périphérique 1310 permet de caler l'embase en direction axiale.

Chaque traversée comprend une broche cylindrique électriquement conductrice 1141 qui s'étend axialement à travers un isolateur 1142.

Les traversées (broches 1141 et isolateurs 1142) sont montées de façon étanche dans des logements complémentaires respectifs 1320 formés dans l'embase, logements où elles sont soudées par tirs laser, ce qui permet de garantir l'étanchéité.

Dans le présent exemple de réalisation, l'embase 1300 portant les traversées 114 est elle-même soudée, par exemple par tirs laser, sur la pièce 1100 formant face avant.

En référence aux Figures 12 et 13, on va maintenant décrire la façon dont les microcâbles 126 de la microsonde 120 (ici au nombre de quatre) sont reliés aux traversées respectives.

Chaque microcâble 126 est constitué, de façon conventionnelle, par un toron revêtu d'une gaine isolante. Chaque toron est préalablement dénudé sur une longueur typiquement de l'ordre de 1 mm, puis une pièce de jonction électrique 1400, de préférence en acier inoxydable, est montée sur cette partie dénudée.

Chaque pièce de jonction 1400 comprend un corps principal généralement cylindrique 1410 traversé par un passage axial 1420 destiné à accueillir la broche 1141 d'une traversée respective 114. Ce corps 1410 est prolongé latéralement, sur une partie de sa longueur axiale, par un appendice 1430 de connexion avec le microcâble associé, traversée par un passage axial 1440 de diamètre choisi en fonction de celui du toron du microcâble 126.

La liaison mécanique et électrique entre la pièce de jonction 1400 et le microcâble associé est assurée en introduisant l'extrémité dénudée du toron dans le passage 1440 et en réalisant un soudage par tir laser de préférence en bout de câble (du côté droit de l'appendice 1430 sur la Figure 12) de manière à ne pas endommager la structure, en particulier le gainage isolant.

La connexion électrique entre les microcâbles 126 et les traversées 114 est finalisée en engageant les pièces 1400 sur les broches respectives 1141, ces dernières étant engagées dans les passages 1420. Cette opération s'effectue en engageant lesdites pièces 1400 dans leur cavité respective 1252 formée dans la pièce 1200, les ouvertures 1254 qui prolongent ces cavités étant alignées avec les broches 1141 et ces dernières faisant saillie dans lesdites ouvertures 1254 et lesdites cavités 1252 à partir de l'embase 1300.

Une fois cette mise en place effectuée, un soudage par tir laser est réalisé au niveau des extrémités libres des broches 1141 (à gauche des pièces 1400 sur les Figures 12 et 13).

Les pièces de jonction 1400 sont alors bloquées dans leurs cavités respectives 1252, et verrouillées selon les six degrés de liberté.

En référence aux Figures 6 et 7, on va maintenant décrire la partie intermédiaire de transition 124 entre le corps 110 de la capsule 100 et la sonde 120, ainsi qu'une partie surmoulée.

Cette partie est ici réalisée sous la forme d'un ensemble de tubes souples par exemple en silicone, ici trois tubes 1241 à 1243, qui sont préassemblés et collés entre eux, ou formés d'un tube unique, puis enfilés par le coté distal de la sonde 120 jusqu'à la zone de jonction et collés sur la sonde. Par sa fonction de gradient de raideur, cette partie de transition 124 permet de disposer d'une résistance mécanique importante dans la zone de jonction formée par les pièces 1100, 1200, 1300 et 1400, telle que décrite dans ce qui précède, en limitant les courbures, et d'une résistance mécanique qui diminue progressivement pour rejoindre, au niveau de la sonde 120, une déformabilité du même ordre que celle de ladite sonde.

On observe également que le tube de silicone 1241 adjacent à la zone de jonction est doté d'un épaulement 1241a (réalisé par exemple d'un seul tenant avec le tube 1241 ou bien constitué par une section de tube de silicone plus large collée au tube 1241) destiné à assurer une retenue mécanique de la partie 124 sur la capsule grâce à une zone surmoulée que l'on va maintenant décrire en référence particulière à la Figure 7.

Cette zone surmoulée, désignée par la référence 1500, est de préférence constituée d'un élastomère thermoplastique de polyuréthane tel que le *Tecothane* (marque déposée), et réalisée par une technique conventionnelle de surmoulage et présente une forme généralement conique de révolution, avec une base large de diamètre de préférence égal à celui de la capsule 110, et une terminaison plus étroite, arrondie à l'extérieur et à l'intérieur de l'extrémité conique pour être atraumatique, qui emprisonne l'épaulement 1241a.

Cette zone surmoulée verrouille en place l'ensemble des pièces de liaison décrites dans ce qui précède, et notamment les pièces de jonction 1400, pour conférer une grande stabilité à l'ensemble et notamment garantir la reprise des efforts mécaniques entre le corps 110 de la capsule 100 et la microsonde 120. Notamment, la résistance aux efforts de traction axiale entre la microsonde et la capsule est assurée à la fois par les soudures laser des microcâbles 126 aux pièces de jonction 1400, par les soudures laser des pièces de jonction 1400 aux broches 114A, et par le blocage mécanique des pièces par la zone surmoulée 1500. Cette dernière assure également le blocage des pièces 1100 et 1200 contre leur rotation inverse, en remplissant les espaces vides au voisinage des languettes 1130 et 1230.

L'ensemble de liaison et de connexion décrit dans ce qui précède est de préférence assemblé par les étapes ordonnées suivantes :
1°) assemblage mécanique de la pièce formant face avant 1100, portant l'embase 1300 pourvue des isolateurs 1142, et de la pièce formant support de liaison 1200 par translation axiale puis rotation, comme décrit plus haut, avec serrage grâce aux pentes des languettes ;
2°) mise en place et ajustement des traversées (broches 1141 et isolateurs 1142) dans leurs logements respectifs 1320, puis fixation par soudage laser par l'arrière ;
3°) engagement des extrémités dénudées des microcâbles 126 dans les pièces de jonction respectives 1400, puis soudage laser ;
4°) engagement des pièces de jonction 1400 sur les broches 1141 puis soudage laser par l'avant ;
5°) mise en place de la pièce de transition 124 autour de la microsonde 120 et collage sur celle-ci ;
6°) encapsulation de l'ensemble par surmoulage de la zone 1500 ;
7°) assemblage des circuits internes à loger dans le corps 110 de la capsule hybride 100 et connexion avec les extrémités des broches 1141 qui font saillie côté arrière, puis mise en place autour de ces circuits du boitier du corps de capsule et fixation de ce dernier sur la pièce formant face avant 1100, par exemple par un cordon de soudure laser. Un épaulement 1140 de la pièce 1100, visible notamment sur la Figure 11, permet d'assurer la mise en place correcte, avec butée, du corps de capsule sur la pièce 1100.

De nombreuses variantes peuvent être apportées à la capsule hybride et aux moyens de liaison qui viennent d'être décrits. En particulier :
- bien que l'on ait décrit et illustré une configuration cylindrique de révolution pour la capsule hybride et son dispositif de liaison comprenant les éléments 1100, 1200, 1300, d'autres formes sont bien entendu possibles (par exemple section ovale, elliptique, polygonale telle que carrée ou rectangulaire - avec alors de préférence des transitions arrondies) ;
- le nombre de traversées à prévoir sera adapté au nombre de pôles d'entrée/sortie de la microsonde ;
- une section rectangulaire aplatie peut être particulièrement adaptée pour le cas où la capsule hybride comprend un système de récupération d'énergie, par exemple du type piézoélectrique ; dans ce cas, les traversées 114 sont avantageusement prévues en ligne ;
- les traversées 114 peuvent être directement intégrées à une face avant du corps de la capsule, l'embase 1300 faisant alors partie intégrante de ce corps.

## Revendications

1. Un dispositif médical implantable actif formé d'une capsule hybride autonome (100) notamment pour implantation sous-cutanée,
ce dispositif comprenant un corps étanche (110) logeant des circuits électroniques et des moyens d'alimentation électrique de ces circuits électroniques et, prolongeant ce corps, au moins une sonde (120) de détection et/ou de délivrance de thérapie, cette sonde comprenant un ensemble de câbles (126) formés chacun d'un câble de coeur électriquement conducteur relié à un pôle respectif desdits circuits électroniques et au moins une électrode de détection et/ou de délivrance de thérapie,
**caractérisé en ce que** ladite sonde (120) est une microsonde comprenant un ensemble de microcâbles,
**en ce que** le dispositif est dépourvu de connecteur électrique entre la microsonde et les circuits électroniques,
**en ce que** chaque câble de coeur (126) est relié à son pôle respectif par des moyens de liaison permanente,
et **en ce que** les moyens de liaison permanente comprennent :
- une embase isolante (1300) traversée de façon hermétique par un ensemble de broches (114) correspondant aux pôles respectifs des circuits électroniques ;
- un support (1100) recevant l'embase de façon hermétique ;
- un ensemble de pièces conductrices de jonction (1400), chaque pièce de jonction comprenant un premier passage (1440) pour un câble de coeur respectif (126) et un second passage (1420) pour une broche respective (114) et étant soudée sur ces derniers ;
- une pièce isolante de guidage et de retenue (1200) montée sur le support (1100) et verrouillée mécaniquement sur celui-ci, ladite pièce de guidage et de retenue comportant un ensemble de cavités (1252) pour les pièces de jonction respectives (1400), chaque cavité s'ouvrant en son fond sur un passage (1254) dans lequel fait saillie une broche respective (114) ; et
- au moins un élément de transition et de protection (1500) encapsulant la pièce de guidage et de retenue, les pièces de jonction et des régions des câbles de coeur qui leur sont attenantes.

2. Le dispositif de la revendication 1, dans lequel le support (1100) fait partie du corps de la capsule.

3. Le dispositif de la revendication 1, dans lequel le support (1100) est constitué d'une pièce formant face avant par laquelle les moyens de liaison (1200, 1300, 1400, 1500) sont fixés sur le corps de la capsule.

4. Le dispositif de la revendication 1, dans lequel la pièce de guidage et de retenue (1200) est montée sur le support par un mouvement de translation suivi d'un mouvement de rotation assurant la mise en correspondance de languettes de verrouillage (1230, 1130) prévues respectivement sur la pièce de guidage et de retenue (1200) et sur le support (1100).

5. Le dispositif de la revendication 4, dans lequel au moins certaines languettes (1230, 1130) forment une rampe assurant une mise en pression des languettes entre elles lors de la rotation.

6. Le dispositif de la revendication 4, dans lequel la pièce de guidage et de retenue (1200) comprend un corps principal (1210, 1220) portant des languettes de verrouillage (1230) et guidant ladite pièce dans le support, et un corps secondaire plus étroit (1250) à la périphérie duquel sont formées les cavités (1252).

7. Le dispositif de la revendication 1, dans lequel les pièces de jonction (1400) comprennent chacune un corps principal (1410) dans lequel est formé le passage (1420) pour la broche respective, et un appendice (1430) s'étendant latéralement par rapport audit passage pour la broche (114) et dans lequel est formé le passage (1440) pour le câble de coeur (126).

8. Le dispositif de la revendication 1, dans lequel l'élément de transition et de protection comprend une région de matériau souple (1500) assurant l'encapsulation et dont la section transversale diminue progressivement du corps (110) de la capsule vers la microsonde (120).

9. Le dispositif de la revendication 1, dans lequel l'élément de transition comprend, autour de la région proximale de la microsonde attenante au corps (110) de la capsule, une région de transition (124) à gradient de raideur variable, décroissant dans le sens distal.

10. Le dispositif des revendications 8 et 9 prises en combinaison, dans lequel la région de transition (124) est formée d'au moins une pièce (1241-1243) distincte de la région d'encapsulation et comprend à son extrémité proximale un aménagement (1241a) de verrouillage dans la région d'encapsulation.

11. Le dispositif de la revendication 1, dans lequel le corps (110) de la capsule présente une forme généralement allongée de section transversale essentiellement constante, et dans lequel les moyens de liaison sont prévus à une extrémité du corps et occupent une section transversale au plus égale à celle du corps.

## Patentansprüche

1. Aktive implantierbare medizinische Vorrichtung in Form einer autonomen Hybridkapsel (100), insbesondere zur subkutanen Implantierung,
wobei diese Vorrichtung einen dichten Körper (110) umfasst, der elektronische Schaltungen und Stromversorgungseinrichtungen dieser elektronischen Schaltungen aufnimmt, sowie, im Anschluss an diesen Körper, mindestens eine Erfassungs- und/oder Therapieverabreichungssonde (120), wobei diese Sonde einen Satz Kabel (126) umfasst, die jeweils aus einem mit einem jeweiligen Pol der elektronischen Schaltungen verbundenen elektrisch leitenden Herzkabel und mindestens einer Erfassungs- und/oder Therapieverabreichungssonde gebildet sind,
**dadurch gekennzeichnet, dass** die Sonde (120) eine Mikrosonde ist, die einen Satz Mikrokabel umfasst,
dass die Vorrichtung zwischen der Mikrosonde und den elektronischen Schaltungen keine elektrische Verbindung aufweist,
dass jedes Herzkabel (126) über permanente Verbindungsmittel mit seinem jeweiligen Pol verbunden ist,
und dass die permanenten Verbindungsmittel Folgendes umfassen:
- einen isolierenden Sockel (1300), der durch einen Satz Stifte (114), die den jeweiligen Polen der elektronischen Schaltungen entsprechen, hermetisch durchquert wird;
- einen Träger (1100), der den Sockel hermetisch aufnimmt;
- einen Satz von leitenden Verbindungsstücken (1400), wobei jedes Verbindungsstück einen ersten Durchgang (1440) für ein jeweiliges Herzkabel (126) und einen zweiten Durchgang (1420) für einen jeweiligen Stift (114) umfasst, und an diese angeschweißt ist;
- ein auf dem Träger (1100) montiertes und an ihm mechanisch verriegeltes isolierendes Führungs- und Rückhaltestück (1200), das einen Satz von Ausnehmungen (1252) für die jeweiligen Verbindungsstücke (1400) aufweist, wobei jede Ausnehmung sich zu einem Durchgang (1254) hin öffnet, in den ein jeweiliger Stift (114) hineinragt; und
- mindestens ein Übergangs- und Schutzelement (1500), welches das Führungs- und Rückhaltestück, die Verbindungsstücke und die daran angrenzenden Bereiche der Herzkabel einkapselt.

2. Vorrichtung nach Anspruch 1, bei der der Träger (1100) Teil des Körpers der Kapsel ist.

3. Vorrichtung nach Anspruch 1, bei der der Träger (1100) aus einem Stück besteht, das eine Stirnfläche bildet, über die die Verbindungsmittel (1200, 1300, 1400, 1500) am Körper der Kapsel befestigt sind.

4. Vorrichtung nach Anspruch 1, bei der das Führungs- und Rückhaltestück (1200) durch eine Translationsbewegung gefolgt von einer Drehbewegung am Träger montiert wird, wodurch für eine Übereinstimmung von jeweils am Führungs- und Rückhaltestück (1200) und am Träger (1100) vorgesehenen Verriegelungslaschen (1230, 1130) gesorgt wird.

5. Vorrichtung nach Anspruch 4, bei der zumindest einige der Laschen (1230, 1130) eine Rampe bilden, die bei der Rotation für einen Druckaufbau zwischen den Laschen sorgt.

6. Vorrichtung nach Anspruch 4, bei der das Führungs- und Rückhaltestück (1200) einen Hauptkörper (1210, 1220) umfasst, der Verriegelungslaschen (1230) trägt und das Stück in den Träger hineinführt, sowie einen engeren Nebenkörper (1250), an dessen Umfang die Ausnehmungen (1252) gebildet sind.

7. Vorrichtung nach Anspruch 1, bei der die Verbindungsstücke (1400) jeweils einen Hauptkörper (1410) umfassen, in dem der Durchgang (1420) für den jeweiligen Stift gebildet ist, sowie einen Fortsatz (1430), der sich seitlich zum Durchgang für den Stift (114) erstreckt und in dem der Durchgang (1440) für das Herzkabel (126) gebildet ist.

8. Vorrichtung nach Anspruch 1, bei der das Übergangs- und Schutzelement einen Bereich aus einem flexiblen Material (1500) aufweist, der für die Verkapselung sorgt und dessen Querschnitt vom Körper (110) der Kapsel zur Mikrosonde (120) hin allmählich abnimmt.

9. Vorrichtung nach Anspruch 1, bei der das Übergangselement um den an den Körper (110) der Kapsel angrenzenden proximalen Bereich der Mikrosonde einen Übergangsbereich (124) mit einem variablen, in distaler Richtung abnehmenden Steifigkeitsgradienten umfasst.

10. Vorrichtung nach den Ansprüchen 8 und 9 in Kombination miteinander, bei der der Übergangsbereich (124) aus mindestens einem vom Verkapselungsbereich verschiedenen Stück (1241-1243) gebildet ist, und an seinem proximale Ende, im Verkapselungsbereich, eine Verriegelungseinrichtung (1241a) umfasst.

11. Vorrichtung nach Anspruch 1, bei der der Körper (110) der Kapsel eine allgemein langgestreckte Form mit einem im Wesentlichen konstanten Querschnitt aufweist, und bei der die Verbindungsmittel an einem Ende des Körpers vorgesehen sind und einen Querschnitt einnehmen, der höchstens dem des Körpers gleicht.

## Claims

1. An active implantable medical device formed of an autonomous hybrid capsule (100), in particular for subcutaneous implantation,
the device comprising a hermetical body (110) housing electronic circuitry and power supply means for powering the electronic circuitry and at least one detection and/or therapy delivery lead (120) extending from the body, the lead comprising a set of cables (126) each formed of an electrically conductive core cable connected to a respective pole of said electronic circuitry and at least one detection and/or therapy delivery electrode,
**characterized in that** the lead (120) is a microlead comprising a set of microcables,
**in that** the device lacks an electrical connector between the microlead and the electronic circuitry,
**in that** each core cable (126) is connected to its respective pole via permanent connection means,
and **in that** the permanent connection means comprise:
- an insulating base (1300) hermetically traversed by a plurality of pins (114) corresponding to the respective poles of the electronic circuitry;
- a support (1100) hermetically receiving the base;
- a set of conductive connection parts (1400), each connection part comprising a first passage (1440) for a respective core cable (126) and a second passage (1420) for a respective pin (114) and being welded to the latter;
- an insulating guidance and retaining part (1200) mounted on the support (110) and mechanically locked thereto, said guidance and retaining part having a plurality of recesses (1252) for the respective connection parts (1400), each recess opening at its bottom onto a passage (1254) into which a respective pin (114) projects; and
- at least one transition and protection element (1500) encapsulating the guidance and retaining part, the connection parts, and adjoining regions of the core cables.

2. The device according to claim 1, wherein the support (1100) is part of the capsule body.

3. The device according to claim 1, wherein the support (1100) consists of a part forming a front face whereby the connection parts (1200, 1300, 1400, 1500) are fixed to the capsule body.

4. The device according to claim 1, wherein the guidance and retaining part (1200) is mounted on the support by a translational movement followed by a rotational movement ensuring the matching of locking tabs (1230, 1130) respectively provided on the guidance and retaining part (1200) and on the support (1100).

5. The device according to claim 4, wherein at least some of the locking tabs (1230, 1130) form a ramp that ensures a pressurization of the locking tabs during rotation.

6. The device according to claim 4, wherein the guidance and retaining part (1200) includes a main body (1210, 1220) having locking tabs (1230) and guiding said part into the support, and a narrower secondary body (1250) at the periphery of which the recesses (1252) are formed.

7. The device according to claim 1, wherein the connection parts (1400) each comprise a main body (1410) in which the passage (1420) for the respective pin is formed, and an extension (1430) extending laterally with respect to the passage for the pin (114) and in which the passage (1440) for the core cable (126) is formed.

8. The device according to claim 1, wherein the transition and protection element comprises a region of flexible material (1500) providing encapsulation and whose cross-section gradually decreases from the body (110) of the capsule towards the microlead (120).

9. The device according to claim 1, wherein the transition element includes, around a proximal region of the microlead adjacent to the body (110) of the capsule, a transition region (124) with a variable stiffness gradient, decreasing in the distal direction.

10. The device according to claims 8 and 9 combined, wherein the transition region (124) is formed of at least one part (1241-1243) distinct from the encapsulation region and comprises at its proximal end a locking arrangement (1241a) in the encapsulation region.

11. The device according to claim 1, wherein the body (110) of the capsule has a generally elongated shape of substantially constant cross-section, and wherein the connection means are provided at one end of the body and occupy a cross-section at most equal to that of the body.
